(19) Europäisches Patentamt European Patent Office Office européen des brevets

(11) **EP 2 841 129 B1**

(12) **FASCICULE DE BREVET EUROPEEN**

(45) Date de publication et mention de la délivrance du brevet:
**11.11.2020 Bulletin 2020/46**

(21) Numéro de dépôt: **13719784.4**

(22) Date de dépôt: **23.04.2013**

(51) Int Cl.:
*A61M 5/142* *(2006.01)*    *A61M 5/168* *(2006.01)*

(86) Numéro de dépôt international:
**PCT/EP2013/058407**

(87) Numéro de publication internationale:
**WO 2013/160312 (31.10.2013 Gazette 2013/44)**

(54) **SYSTEME DE BOUCLE FERMEE DE CONTROLE DU REFLUX D'UNE INJECTION FLUIDIQUE**

GESCHLOSSENER REGELKREIS ZUR STEUERUNG DES RÜCKFLUSSES EINER FLÜSSIGKEITSEINSPRITZUNG

CLOSED LOOP SYSTEM FOR CONTROLLING THE REFLUX OF A FLUID INJECTION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **23.04.2012 FR 1253709**

(43) Date de publication de la demande:
**04.03.2015 Bulletin 2015/10**

(73) Titulaire: **Commissariat à l'Énergie Atomique et aux Énergies Alternatives 75015 Paris (FR)**

(72) Inventeurs:
• **CAMPAGNOLO, Raymond 38100 Grenoble (FR)**
• **JALLON, Pierre 38100 Grenoble (FR)**

(74) Mandataire: **Brevalex 95, rue d'Amsterdam 75378 Paris Cedex 8 (FR)**

(56) Documents cités:
**US-A1- 2005 075 624    US-A1- 2005 145 008
US-A1- 2011 144 540    US-A1- 2011 257 593**

**Description**

## DOMAINE TECHNIQUE ET ART ANTÉRIEUR

L'invention se rapporte à la maîtrise de l'injection localisée d'un fluide, par exemple un fluide médical et/ou thérapeutique.

C'est le cas notamment de l'injection d'un principe thérapeutique dans le parenchyme cérébral pathologique (neuro-dégénératif ou tumoral).

Elle permet la détection d'une amorce de reflux, puis mettre fin à cette amorce et d'assurer ainsi la sécurité d'un patient. Dans le cas contraire, en effet, des effets secondaires peuvent se produire, qui peuvent être délétères, effet, notamment, qui peuvent être liés à la contamination du LCR (ou Liquide Céphalo Rachidien).

Le document US 7 794 443 décrit un dispositif de délivrance localisée d'un principe actif comportant un réservoir, une pompe et un cathéter, le tout associé à un capteur de pression, à un processeur et à une librairie de profil de pression. Il analyse l'évolution temporelle de la pression et la compare à une base de cas (une bibliothèque de profils pré enregistrés) pour déterminer l'état de l'infusion. Un système d'affichage présente à l'opérateur la courbe temporelle de la pression et indique si la délivrance est satisfaisante ou s'il existe un problème du type reflux, de bouchage, de présence de bulle d'air ou de rupture du chemin fluidique.

Dans ce document, l'algorithme de sélection de l'état de délivrance ne tient pas compte des phénomènes dynamiques affectant la pression mesurée et ne se base que sur l'état d'équilibre final obtenu. En cas de reflux il se contente de signaler que l'injection s'est mal déroulée, sans aucune anticipation ni action préventive. C'est donc un système lent, en boucle ouverte : la mesure de la pression n'interagit pas sur le fonctionnement de la pompe. Les mesures sont utilisées a posteriori pour surveiller la délivrance, par exemple en stoppant manuellement l'injection. Cette mesure ne sert pas pour agir automatiquement sur la pompe, en cours de délivrance. En outre, seul l'opérateur peut intervenir sur le déroulement de l'expérience. Les courbes présentées dans ce document ont été obtenues sur un cerveau d'animal sain (ovin), donc la question de l'hétérogénéité des tissus pathologiques n'est pas abordée, ni la notion de comportement dynamique du système.

Un autre document, US 4 534 756, décrit un système d'analyse de la pression associé à une pompe d'infusion et à un organe de contrôle pour détecter des dysfonctionnements (tels qu'une infiltration, et/ou l'état ouvert d'un circuit fluidique, et/ou la présence de bulles d'air) dans l'injection d'une substance active, en mode parentéral, afin de générer une alarme.

Mais ce document ne résout pas les problèmes de reflux.

Le document EP1980201 décrit les différentes causes, dont cette fois l'hétérogénéité des tissus tumoraux, affectant la qualité de la délivrance de liquide thérapeutique pour le traitement du glioblastome. En particulier, la constitution du cathéter et les paramètres tels que la conductivité hydraulique des tissus sont mis en avant, et un modèle de la propagation du traitement dans le parenchyme cérébral est présenté. La procédure d'implantation du cathéter est citée comme une cause possible d'initiation du reflux (création d'un inter-espace autour du cathéter qui favorise l'échappement du liquide par sa faible impédance hydraulique). Un logiciel permet l'aide, par imagerie IRM, au placement du cathéter, à bonne distance de cavités ou de structures telles que les ventricules ou les sillons cérébraux risquant d'entrainer une fuite du principe thérapeutique réduisant ainsi presque à néant l'efficacité de la thérapie anti-tumorale.

L'article « Focal delivery during direct infusion to brain : role of flow rate, cathéter diameter and tissue mechanics » de Paul F. Morrison et al., pose les bases d'un modèle mathématique de la délivrance d'un principe actif à partir d'un cathéter et donne des exemples de reflux survenus suite à un débit de liquide incompatible avec les propriétés hydraulique (conductivité) des tissus cérébraux. Les autoradiographies affichées dans l'article montrent que, à faible débit, le reflux est négligeable mais qu'aux environs de $5\mu l/min$, il y a une fuite du liquide vers des structures du cortex du rat.

Le document US 2005/075624 décrit une mesure de pression dans des dispositifs médicaux implantables.

Le document US 2011/0257593 décrit des dispositifs et procédés pour détecter des complications dans un cathéter.

Le document US 2011/0144540 décrit une surveillance des conditions d'un dispositif médical implantable pour délivrer des fluides.

Le document US 2005/0145008 décrit un système de détection du statut d'un évent associé à l'alimentation en fluide en amont d'une pompe à infusion.

## EXPOSÉ DE L'INVENTION

On décrit d'abord un système d'injection et de contrôle d'un flux de liquide à injecter, selon la revendication 1.

En particulier les moyens de contrôle peuvent permettre de détecter une situation de reflux lors d'une injection et d'agir sur le fonctionnement de la pompe en inversant son fonctionnement, lorsque la pompe est réversible, c'est-à-dire qu'elle permet d'aspirer le fluide présent dans le cathéter. Un tel système est capable de réagir rapidement à l'amorce d'un reflux le long d'un cathéter par l'analyse du comportement dynamique de la pression mesurée par un capteur.

Dans un exemple, un tel système peut être utilisé pour la distribution d'un principe thérapeutique dans un tissu pathologique, par exemple dans une tumeur cérébrale.

Fréquemment, lors de la délivrance d'un fluide dans un tissu, la pression dans le cathéter augmente peu à peu, jusqu'à atteindre un équilibre.

Le capteur de pression permet de mesurer l'évolution de cette dernière en fonction du temps. Les moyens de contrôle déterminent un indicateur basé sur une mesure de cette pression à des instants prédéterminés. Par exemple, cet indicateur est une combinaison de différentes pressions observées. Il peut notamment s'agir d'une comparaison entre deux mesures de pressions, sous la forme d'une différence ou d'un ratio.

Généralement, une pompe péristaltique fonctionne par cycles, au cours desquels le fluide est comprimé, chaque cycle permettant alors la délivrance d'un volume élémentaire de fluide. Les moyens de contrôle établissent un indicateur, associé ou relatif à un même cycle, à partir, ou en fonction, d'au moins deux mesures de la pression à différents instants d'un même cycle.

Les moyens de contrôle sont alors aptes à inverser la pompe en fonction de la valeur de cet indicateur.

Les moyens de contrôle sont aptes à générer un indicateur en fonction de la comparaison de la pression mesurée à deux instants d'un même cycle, notamment en fonction de la différence entre les pressions mesurées à deux instants d'un même cycle.

En fonction de la valeur de l'indicateur, les moyens de contrôle agissent sur la pompe, ils sont notamment aptes à inverser le sens d'écoulement du fluide. Cela peut se faire en comparant l'indicateur à une valeur seuil prédéterminée.

On peut ainsi détecter, au plus tôt, et interdire, l'apparition puis la propagation d'un phénomène de reflux, avant qu'il ne devienne impossible à maîtriser (par exemple une contamination du Liquide Céphalo Rachidien), l'objectif étant de stopper au plus vite la délivrance du produit, voire d'aspirer une partie du liquide. Ainsi, l'indicateur décrit ci-dessus peut témoigner de la présence ou de l'absence d'un reflux.

Des moyens de contrôle du débit peuvent être prévus, pour augmenter ou réduire le débit de la pompe en fonction de la valeur de l'indicateur.

Un tel système peut également permettre de faire croître, par paliers successifs, le débit de la pompe jusqu'à une consigne déterminée par l'utilisateur, tout en disposant d'un mécanisme de sécurité qui limitera automatiquement ce débit dès l'apparition d'une amorce de reflux. Cela permet de limiter les conséquences de l'apparition d'un éventuel reflux.

Le système participe donc à une augmentation de la sécurité de l'acte de délivrance thérapeutique.

Si une maîtrise complète du reflux ne peut être obtenue, par exemple du fait d'une très grande hétérogénéité locale de la structure du tissu (par exemple encore dans le cas du parenchyme cérébral pathologique), ce système permet l'arrêt le plus rapide possible de la délivrance et donc la contamination la plus minime des structures du tissu ou de l'organe concerné (dans l'exemple : le cerveau).

Ce système peut être doté d'une interface de communication pour transmettre à l'utilisateur l'état du système, lors d'une détection d'une amorce de reflux, ou d'une réduction du débit, ou d'un débit réellement injecté ou d'un arrêt de la délivrance suite à une situation critique. Cela est réalisé automatiquement, et non manuellement comme dans le brevet US 7794443.

Le capteur de pression peut être disposé entre la pompe et l'extrémité du conduit, par laquelle est délivré le fluide.

La pompe péristaltique peut être par exemple une micro-pompe péristaltique ; dans un mode particulier, celle-ci peut être réalisée en technologie silicium. En variante, ce peut être une micro-pompe à vannes passives.

Un cycle de pompage peut comporter :

- une première phase, durant laquelle la pompe ne délivre pas de liquide dans le cathéter, la pression dans le cathéter étant stable en l'absence de reflux ;
- une deuxième phase, durant laquelle la pompe injecte le liquide dans le cathéter, induisant un saut de pression dans le cathéter, la présence d'un reflux se traduisant par un amoindrissement de ce saut de pression, par rapport à un saut de pression en l'absence de reflux.

Le liquide à injecter est par exemple un liquide médical tel qu'un liquide antitumoral.

## BRÈVE DESCRIPTION DES DESSINS

On décrira à présent, à titre d'exemples non limitatifs, des modes de réalisation de l'invention, en se référant aux dessins annexés, dans lesquels :

- la figure 1 représente un système pouvant être mis en œuvre dans le cadre la présente invention ;
- les figures 2A-2C représentent le dispositif de la figure 1, et des variantes de ce dispositif, à chaque fois dans une utilisation spécifique ;
- la figure 3 représente un exemple de micro-pompe pouvant être utilisée dans le cadre de la présente invention ;
- les figures 4A et 4B représentent respectivement l'évolution de la pression lors de l'injection d'un fluide, sans reflux, et une visualisation de l'injection ;
- les figures 5A et 5B représentent respectivement l'évolution de la pression lors de l'injection d'un fluide, avec reflux, et une visualisation de l'injection ;
- la figure 6 représente un agrandissement du profil de pression de la figure 5A ;
- les figures 7A-7G représentent des étapes de fonctionnement d'une micro-pompe utilisée dans le cadre d'une réalisation de la présente invention ;
- les figures 8A-8C représentent des courbes de mesure de pression, superposées au cycle de fonctionnement d'une micro pompe, illustrant le cycle de fonctionnement d'une micro pompe (figure 8A), l'évolution de la pression en cas d'injection normale (figure 8B) et l'évolution de la pression en cas de reflux (figure 8C).

- les figures 9A-9B représentent l'évolution d'un paramètre de fonctionnement de la pompe, dans un cas ou il n'y a pas de reflux (figure 9A) et dans un cas de reflux (figure 9B).

## EXPOSÉ DÉTAILLÉ DE MODES DE RÉALISATION PARTICULIERS

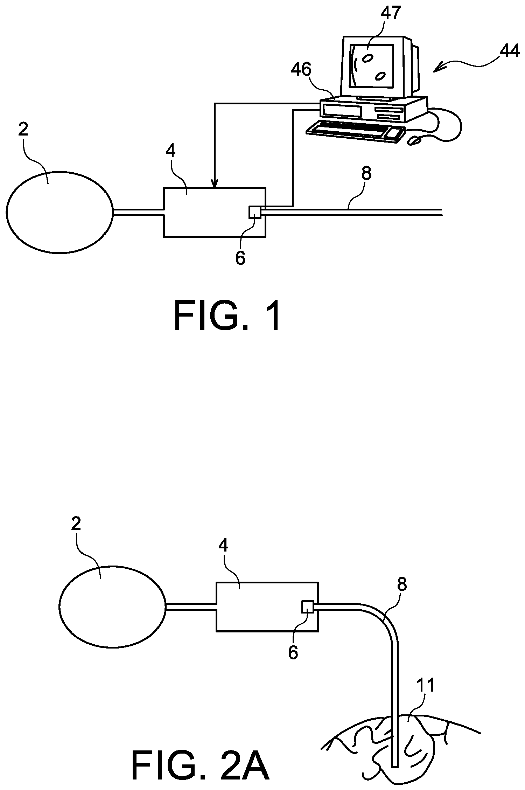

Un système représenté en figure 1 comporte une pompe péristaltique 4, pour contrôler l'injection d'un fluide par l'intermédiaire d'un cathéter 8. Ce fluide provient lui-même d'un réservoir 2, relié à la pompe péristaltique.

Une pompe péristaltique permet de pouvoir changer d'état le plus rapidement possible. Elle possède des vannes d'entrée et de sortie actives (pilotables électriquement). C'est par exemple une pompe MEMS à actionnement de type piézo-électrique. Un exemple de micro-pompe pouvant être utilisée dans le cadre de la présente invention est décrite dans le document WO2011/058140 et sera décrit un peu plus en détail ci-dessous.

De préférence, la pompe a un fonctionnement réversible : elle peut donc gérer, de la même manière, une distribution de liquide, depuis le réservoir 2, vers un tissu, par exemple un tissu du corps humain ou d'un animal, notamment le cerveau, et la récupération d'un liquide en phase d'injection, depuis le tissu vers le réservoir. Cette faculté autorise la diminution de la pression en sortie du cathéter en un temps très court. Le repompage d'une fraction du liquide, contenu dans le cathéter 8, permet non seulement de bloquer la propagation d'un reflux le long de ce même cathéter, mais également de repomper tout ou partie du liquide formant le reflux. En effet, puisqu'un chemin de faible résistance hydraulique est en cours de création à la surface externe du cathéter, le pompage inverse évacue la fraction de liquide contenu dans le cathéter, puis cette poche de liquide, avant de débuter le retrait du liquide présent dans les tissus. Cela évite la délivrance d'un liquide dans un endroit non désiré, ce qui est un avantage particulièrement important lorsqu'on souhaite délivrer un liquide dans des organes tels que le cerveau.

L'adhérence entre le cathéter et le tissu initialement en contact peut ensuite être rétablie si on laisse suffisamment de temps au système pour trouver un nouvel état d'équilibre.

Selon un mode de réalisation, le débit de pompage, c'est-à-dire le débit du fluide délivré, initialement nul, est augmenté peu à peu. Le fait de constater un reflux permet une estimation du débit maximal $Q_{max}$ correspondant au débit de la pompe lorsque le reflux apparaît. On comprend alors que le fait de détecter un reflux permet d'ajuster le débit de pompage Q à une valeur inférieure au débit $Q_{max}$, ce dernier correspondant à la valeur du débit ayant entraîné un reflux.

Le cathéter 8 est de préférence un cathéter souple et adhérent aux tissus.

Un capteur de pression 6 est associé au dispositif. Comme illustré sur les différentes variantes des figures 2A - 2D, ce capteur peut être positionné en différents endroits du système.

En figure 1 (et en figure 2A), le capteur est intégré dans la micro pompe 4. Une réalisation d'une micro pompe en silicium permet d'inclure directement le capteur de pression dans le substrat de la micro-pompe, comme décrit dans la demande WO2011/058140.

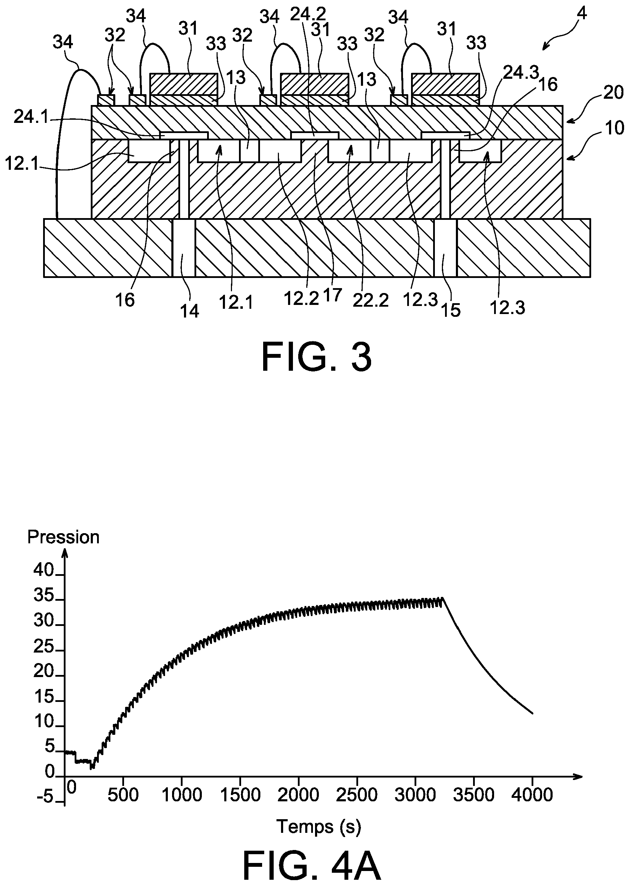

Une telle pompe 4 peut avoir la structure de la figure 3, comportant un premier substrat 10 et second substrat 20 formés, par exemple, en silicium ou en verre. Ils peuvent avoir été assemblés par scellement moléculaire ou par scellement anodique.

Au moins 3 zones du second substrat 20 sont situées en regard d'au moins 3 cavités 12-1, 12-2, 12-3 réalisées dans le premier substrat 10 pour former des membranes déformables 22-1, 22-2, 22-3. Ces cavités sont connectées en série par l'intermédiaire des conduits de communication 13. La cavité centrale 12-2 contribue à former la chambre de pompage et les deux cavités amont 12-1 et aval 12-3 contribuent à former des vannes actives.

Des conduits d'entrée 14 et de sortie 15 qui traversent le premier substrat 10 sont réalisés sous forme de puits débouchants à l'intérieur, respectivement, des cavités amont 12-1 et aval 12-3.

Les conduits d'entrée 14 et de sortie 15 débouchent dans lesdites cavités par un orifice bordé d'une lèvre annulaire 16. Les lèvres 16 peuvent présenter une hauteur sensiblement égale à la profondeur des cavités dans lesquelles elles sont situées.

Au moins 2 dégagements 24-1, 24-3 peuvent être réalisés dans la face inférieure du second substrat 20, et viennent en regard des lèvres correspondantes 16. Ils permettent de garantir, lors de l'assemblage des substrats, que le sommet des lèvres 16 ne touche pas la face inférieure du second substrat 20.

De plus, ces dégagements 24-1, 24-3 assurent également une communication fluidique, dans le cas d'une membrane non contrainte mécaniquement, entre les conduits d'entrée 14 et de sortie 15 et les cavités 12-1, 12-3 dans lesquelles ils débouchent.

Un bossage 17 peut être réalisé dans la face supérieure du premier substrat 10 et localisé sensiblement au centre de la cavité centrale 12-2. Pour éviter le contact entre le sommet du bossage 17 et la face inférieure du second substrat 20, un dégagement 24-2 est avantageusement réalisé dans cette dernière.

En variante, il est possible de ne pas réaliser de dégagements dans la face inférieure du second substrat 20, qui reste alors plane. La hauteur des lèvres 16 est alors inférieure à la profondeur des cavités amont 12-1 et aval 12-3 dans lesquelles elles sont situées. Ainsi, le sommet des lèvres 16 ne touche pas la face inférieure du second substrat et la communication fluidique, dans le cas d'une membrane non contrainte mécaniquement, entre les conduits d'entrée 14 et de sortie 15 et les cavités 12-1, 12-3 dans lesquelles ils débouchent, est également assurée. De la même manière, il est possible de réaliser un bossage 17 dont la hauteur est inférieure à la profondeur de la cavité centrale 12-2 dans lequel il est situé.

Les moyens d'actionnement de ces membranes peuvent être des moyens piézoélectriques 31, positionnées sur des éléments conducteurs 33. Des plots de contact 32, des pistes conductrices et des fils conducteurs 34 permettent d'assurer l'alimentation électrique de la micropompe avec le système extérieur.

Des jauges de contrainte permettent de mesurer la pression selon la présente invention, via la mesure de la déformation des membranes, comme expliqué dans WO 2011/058140. Elles peuvent être réalisées sur la face supérieure du second substrat et disposées au-dessus des membranes déformables. Ces jauges permettent de mesurer la déformation des membranes afin de connaître leur position (position haute, basse ou intermédiaire), et/ou de mesurer des pressions locales dans le microcanal de la micropompe. Il est par exemple possible de mesurer la différence de pression entre la cavité amont et la cavité aval, et ainsi de mesurer le débit de fluide ou détecter une fuite. Dans le cas où le capteur n'est pas intégré dans la micro pompe, ce peut_être un capteur de pression de type déjà connu, disposé au niveau ou en aval du canal de sortie.

Les jauges de contrainte peuvent être réalisées en un matériau conducteur présentant un facteur de jauge élevé, par exemple le métal, tel que le platine, ou, de façon préférée, en un matériau semi-conducteur dopé tel que, par exemple, le silicium dopé p obtenu par implantation d'ions bore. L'implantation d'ions bore peut-être réalisée directement sur la membrane Si.

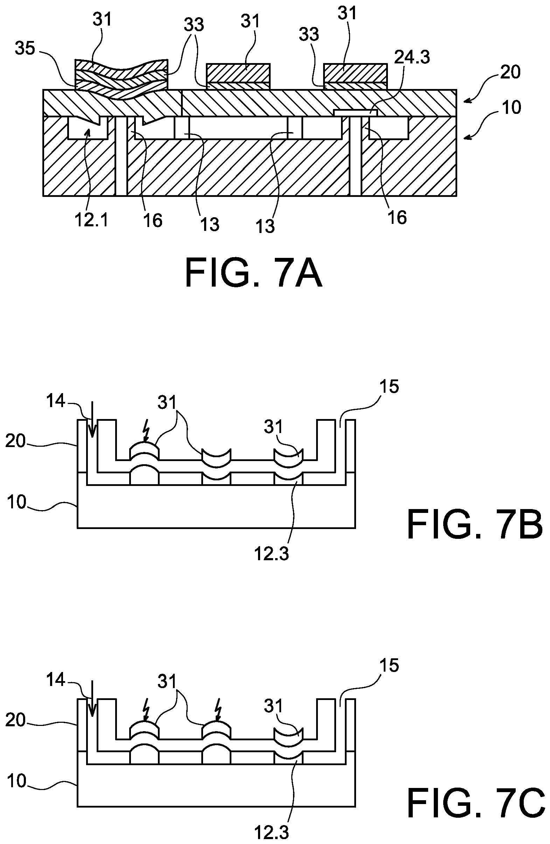

D'autres détails (dimensions, procédés de réalisation) concernant cet exemple de micro-pompe sont donnés dans le document WO 2011/058140. Une variante de la micro pompe présentée ci-dessus est en outre décrite plus loin, en liaison avec la figure 7A, des détails de cette variante pouvant également être trouvés dans le document WO 2011/058140.

Dans tous les cas (celui de la figure 1 mais aussi dans les figures 2A - 2C), le capteur 6 peut être relié à un dispositif 44 de contrôle, pour collection et de traitement des données. Un dispositif de contrôle peut alors contrôler le fonctionnement de la pompe 4, notamment de vannes d'entrée et de sortie, en fonction desdites données.

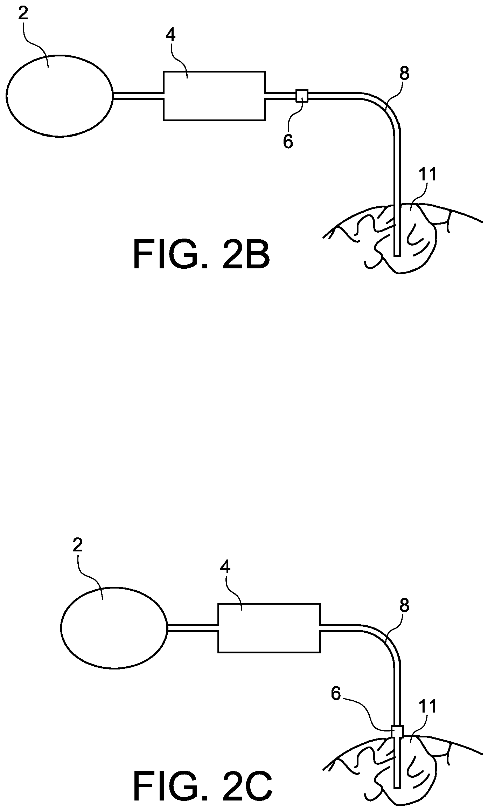

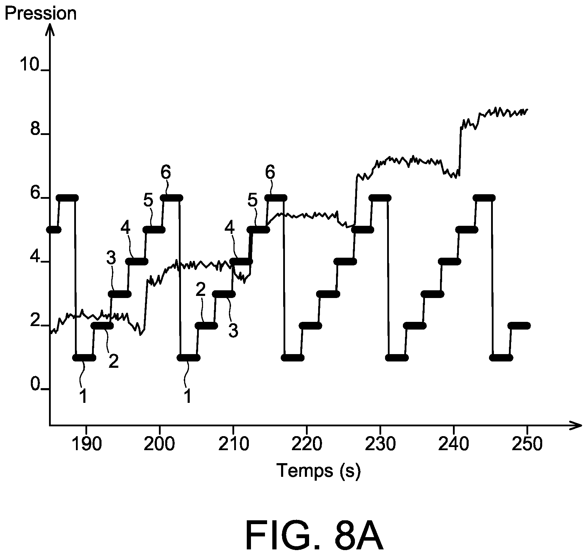

Des moyens électroniques tels qu'un micro ordinateur ou un calculateur, comportant une unité centrale 46, peuvent donc être programmés pour mémoriser et traiter les données du capteur 6, par exemple les données de pression dont il est question plus loin en liaison avec les figures 8A-8C. Cette unité peut être programmée pour mettre en œuvre un procédé de traitement de données selon l'invention. Des moyens 47 d'affichage ou de visualisation permettent, après traitement, de représenter des courbes d'évolution de pression, par exemple du type illustré en figures 4A, 5A ou 6. Les mêmes moyens 44 peuvent mis en œuvre avec les autres configurations du dispositif, représentées en figures 2B - 2C.

Les figures 2A - 2C représentent des variantes du système présenté ci dessus, dans une application de chacun de ces systèmes à l'injection d'un fluide dans un tissu 11, et notamment dans le cerveau. Dans tous les cas, l'extrémité du cathéter 8 est implantée dans le tissu concerné, et le capteur 6 permet de détecter toute variation de pression de l'écoulement du fluide dans le conduit fluidique formé par ce cathéter 8.

Le système représenté en figure 2A est le même que celui de la figure 1, mais lors d'une utilisation pour une injection d'un fluide dans un tissu 11 d'un patient à l'aide du cathéter 8.

En figure 2B, le capteur 6 est positionné en sortie de la micro-pompe, mais dans une portion amont du cathéter 8. Là encore, le capteur peut être réalisé en technologie silicium. Disposée au voisinage de la micro pompe, il peut être intégré dans un boîtier commun à celle-ci.

En figure 2C, le capteur 6 est positionné dans une portion aval du cathéter 8, mais avant l'extrémité de celui-ci. C'est donc un capteur externe, qui peut être situé à l'interface entre les tissus 11 et le cathéter 8. Dans certains cas, il peut en effet être intéressant de placer le capteur de pression plus près de la sortie du cathéter 8, mais en dehors des tissus, par exemple juste sur ou sous la peau.

Ainsi, quel que soit le mode de réalisation, on comprend que le capteur 6 est positionné, de préférence entre la pompe et l'extrémité du cathéter, par laquelle le fluide est délivré. Autrement dit, le capteur 6 est positionné en aval de la pompe.

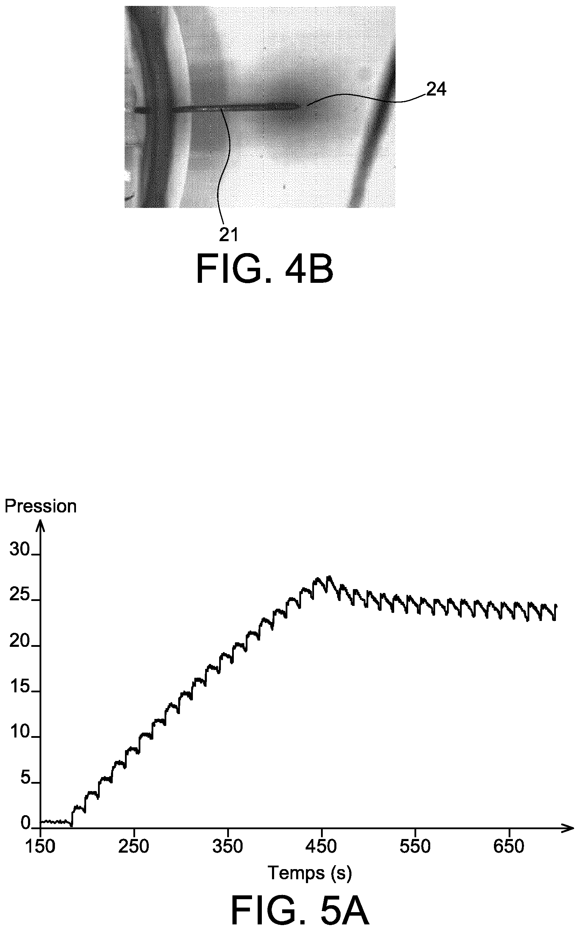

La courbe de la figure 4A représente un enregistrement temporel de l'évolution de la pression à la sortie d'une micro-pompe actionnée par moyens piézoélectriques, avec une aiguille métallique 21 de petit diamètre et en absence de reflux. Sur la figure 4B on voit bien l'aiguille 21 et le milieu 22 dans lequel son extrémité est plongée.

Les paramètres utilisés pour cette mesure sont les suivants :

- une aiguille 32 Gauge (Le Gauge est une unité de mesure utilisé pour le diamètre des aiguilles) ;
- le liquide injecté est un gel d'agarose à 0,6 % ;
- la profondeur d'injection et de 5 mm ;
- le volume d'infusion est de 10 $\mu$L;
- le débit d'injection est de 0,2 $\mu$L/mn ;
- la pompe est une pompe de type MEMS, à actionnement piézo-électrique, à une fréquence de 0,025Hz, sous 170 Vpp (Volt crête - crête).

La courbe de la figure 4A comporte 2 parties :

- une montée en pression, qui va sensiblement de l'origine jusqu'à environ 3200 secondes,
- puis une décroissance de pression qui correspond à l'arrêt de la pompe.

La représentation de la figure 4B permet de visualiser l'injection à l'aide d'un colorant 24. Cette vue confirme l'absence de reflux, une distribution quasi sphérique du colorant ayant été obtenue.

Dans le cas d'un reflux, l'évolution de la pression en fonction du temps montre une décroissance locale de la pression comme l'illustre la représentation de la figure 5A.

Les paramètres utilisés pour cette mesure sont les mêmes que pour la mesure précédente, sauf pour les deux derniers :

- le débit d'injection est de 0,5 $\mu$L/mn ;
- la pompe est une pompe de type MEMS, à actionnement piézo-électrique, à une fréquence de 0,07Hz, sous 170 Vpp.

La courbe de cette figure 5A comporte deux parties :

- une montée en pression, qui va sensiblement de l'origine jusqu'à environ 450 secondes,
- puis une lente décroissance de la pression.

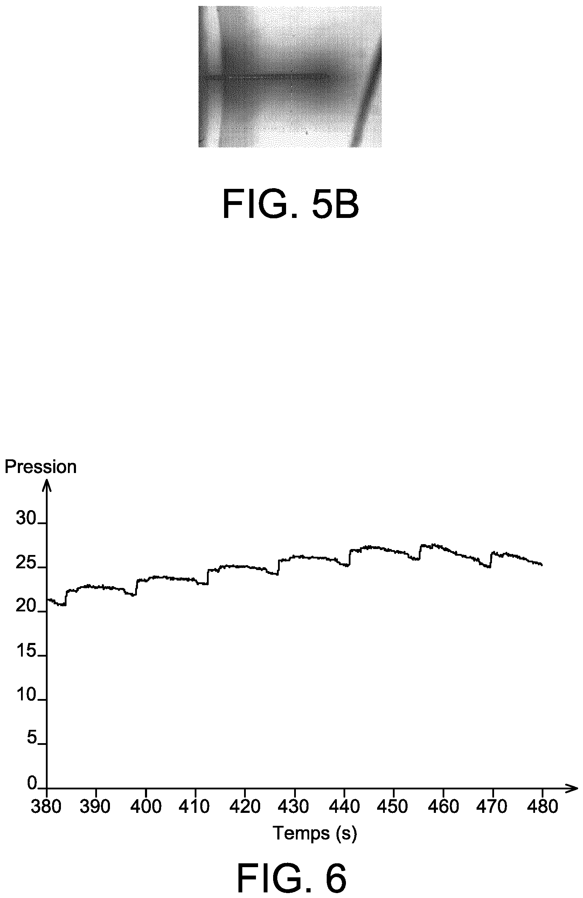

La représentation de la figure 5B permet encore de visualiser l'injection à l'aide d'un colorant 24. Cette vue monte une distribution du colorant localisée autour de l'aiguille, mais avec une forme éloignée de la forme quasi sphérique de la distribution observée en figure 4B. Autrement dit, cette figure correspond à l'apparition d'un reflux.

La figure 6 (pour laquelle les conditions de mesure sont identiques à celles mentionnées ci-dessus pour les figures 5A et 5B) est un zoom sur la période d'apparition du reflux, entre 380 s et 480 secondes. Elle montre qu'à chaque cycle de pompage correspond une impulsion de débit ; ainsi, chaque cycle de pompage se traduit par une impulsion de la pression mesurée au niveau ou en aval de la pompe.

La pompe 4, utilisée dans les mesures présentées ci-dessus, fonctionne en période de 6 phases selon le mode opératoire illustré en figures 7A - 7F.

On a représenté en figure 7A une phase du fonctionnement d'une variante d'une pompe telle que celle décrite ci-dessus en liaison avec la figure 3.

Dans cette variante, une couche contrainte 35 (figure 7A) est déposée directement à la surface de la face supérieure d'au moins une des membranes réalisées, avant le dépôt de la couche conductrice.

Cette couche contrainte exerce un effort sur la membrane concernée qui entraîne une déformation de celle-ci. Par exemple, cette couche contrainte peut être déposée sur les membranes amont et aval (face aux cavités 12.1 et 12.3) et provoque ainsi la mise en contact des membranes avec les lèvres en regard. Aussi, lorsque les membranes ne sont pas activées par les moyens d'actionnement, ici par les pastilles piézoélectriques, les membranes sont déformées dans une position de repos. De cette manière, elles forment des vannes amont et aval fermées au repos. Les autres références numériques de la figure 7A désignent des éléments identiques à ceux qui ont déjà été décrits ci-dessus en liaison avec la figure 3 avec les mêmes références numériques.

La couche contrainte 35 peut être, par exemple, du Si3N3 déposé par PECVD présentant une contrainte interne en tension de l'ordre de plusieurs centaines de méga pascal, par exemple 700 MPa. Son épaisseur peut être de l'ordre de 0,1 $\mu$m à 1 $\mu$m. Comme l'illustre la figure 7A, la déflexion de la membrane 22-1 induite par la couche contrainte 35 est alors de quelques microns et suffit à provoquer la mise en contact de la membrane avec la lèvre 16 en regard.

En figure 7A, on a représenté la première phase de fonctionnement de la pompe, cette phase serait similaire pour la pompe décrite ci-dessus en liaison avec la figure 3. Durant cette phase, le fluide entre alors par le canal 14 et en sort par le canal 15.

On a représenté, en figure 7B - 7F, différentes phases (respectivement phases 1-6) de fonctionnement d'une pompe péristaltique. Sur ce schéma, la pompe est représentée de manière très schématique, avec un conduit d'entrée 14 et un conduit de sortie 15. Ces derniers sont placés de manière différente par rapport à leurs positions dans les structures des figures 3 et 7A, mais cela ne change rien au fonctionnement du dispositif. On a également représenté les moyens d'activation piézo-électrique 31 des différentes chambres.

La phase de la figure 7B (phase 1), au cours de laquelle les moyens 31 d'activation de la chambre 12.1 sont activés, correspond à celle qui est représentée en figure 7A, le fluide entrant alors par le canal 14.

S'ensuivent ensuite les phases, respectivement des figures 7C - 7G :

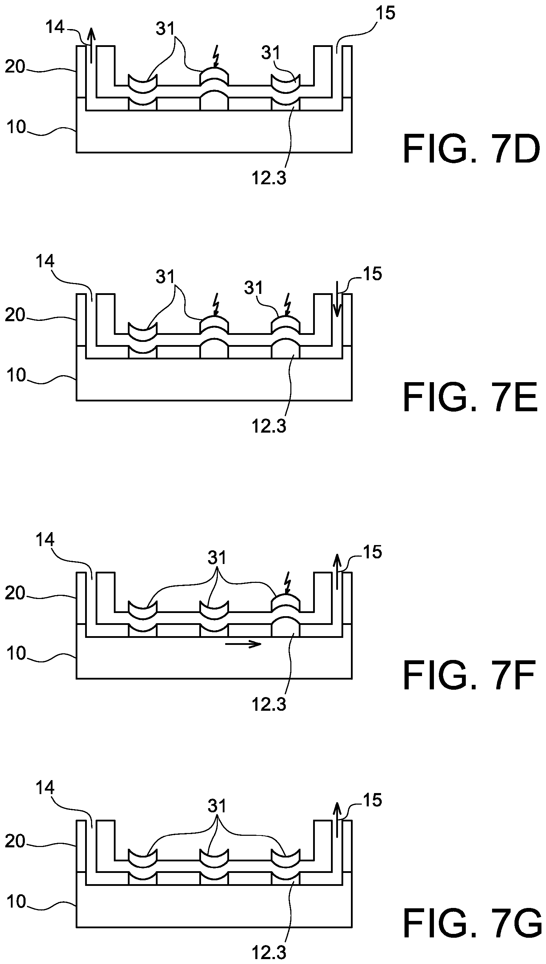

- activation des moyens 31 d'activation des chambres 12.1 et 12.2 (figure 7C, phase 2); le fluide entre alors par le canal 14 et circule de la première chambre 12.1 vers la deuxième chambre 12.2 ;
- activation des moyens 31 d'activation de la chambre 12.2 (figure 7D, phase 3); une partie minime du fluide est refoulé par le canal 14 ; Cette étape permet de former le volume de fluide à délivrer au cours d'un cycle de pompage ;
- activation des moyens 31 d'activation des chambres 12.2 et 12.3 (figure 7E, phase 4); une partie minime de fluide est aspirée du canal 15 ;
- activation des moyens 31 d'activation de la chambre 12.3 (figure 7F, phase 5); Le volume de fluide à délivrer est refoulé dans le canal 15 ;
- désactivation de tous les moyens 31 d'activation des chambres 12.1-12.3 (figure 7G, phase 6) ; le fluide continue à être encore refoulé par le canal 14. Cela achève le refoulement du fluide dans le canal 15.

Ainsi, le fonctionnement de cette pompe est cyclique, une quantité déterminée de fluide étant délivrée (pompée) au cours de chaque cycle.

Les pompes péristaltiques présentent généralement un tel fonctionnement cyclique.

Dans une pompe telle que décrite ci-dessus, la pression peut être mesurée en sortie de la pompe, c'est-à-dire que le capteur 6 de pression se situe en aval de la vanne 12.3. Ajoutons que, dans la pompe qui a été décrite ci-dessus, en liaison avec la figure 3, le capteur permet de mesurer les pressions dans les chambres. Dès lors que la vanne de sortie est ouverte et que la vanne d'entrée est fermée, il permet de mesurer la pression dans le cathéter.

Lorsque la troisième vanne 24.3 est fermée (c'est le cas lors des phases 1-2-3 et 6 qui correspondent, respectivement, aux figures 7A, 7B, 7C et 7G ci-dessus), la pression mesurée est celle dans le cathéter. En l'absence de reflux, la pression évolue peu car la vanne est fermée. Par contre, en présence d'un reflux, cette pression diminue lors de ces phases.

Lors des phases 4 et 5 (qui correspondent aux figures 7E et 7F ci-dessus), la vanne 12.3 est ouverte, la pression mesurée augmente brusquement du fait de l'injection du volume dans le cathéter.

L'augmentation de la pression est généralement plus marquée en l'absence de reflux.

Ainsi, d'une façon générale, un cycle de pompage comporte :

- une première phase, dite phase de repos durant laquelle la pompe ne délivre pas de liquide dans le cathéter : durant cette phase, la pression dans le cathéter est stable en l'absence de reflux, et peut diminuer en présence d'un reflux. En effet, durant cette phase, en l'absence de reflux, il n'y a pas d'écoulement de fluide dans le cathéter. La présence d'un reflux correspond à une fuite, ce qui peut se traduire par une décroissance de la pression ;
- une deuxième phase, durant laquelle la pompe injecte le liquide dans le cathéter, ce qui induit un saut de pression dans le cathéter. La présence d'un reflux peut se traduire par un amoindrissement de ce saut de pression, par rapport à au saut de pression en l'absence de reflux.

Ainsi, on comprend qu'en mesurant une pression à des instants différents au cours d'un cycle, on peut établir un indicateur, dit indicateur de reflux, à partir duquel on peut conclure à la présence ou à l'absence d'un reflux. De préférence, on établit un indicateur relatif à chaque cycle.

Dans cet exemple, cet indicateur est une comparaison de la pression mesurée entre deux instants d'un même cycle. On va alors montrer que la présence d'un reflux diminue la variation de pression entre la fin de la période dite de repos et le début de la période de pompage.

Le cycle de pompage s'observe sur les courbes de mesure du capteur de pression, telles que celles des figures 8A - 8C, sur lesquelles les différentes phases 1 - 6 des premiers cycles sont identifiées. On a également représenté l'évolution de la pression (en unité arbitraire) mesurée au cours de la succession de plusieurs cycles. Comme décrit précédemment, cette pression évolue sensiblement par paliers croissants.

On voit bien, sur la courbe de la figure 8A, que, juste après le début de la phase 5, le liquide est injecté par la pompe 4, ce qui se traduit par une augmentation de la pression mesuré par le capteur 6. Au début de la phase 6, le capteur mesure à nouveau la pression extérieure à la pompe, c'est-à-dire dans le cathéter :

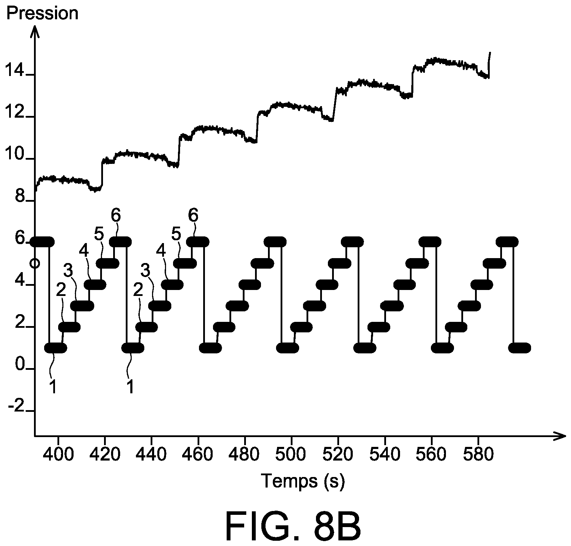

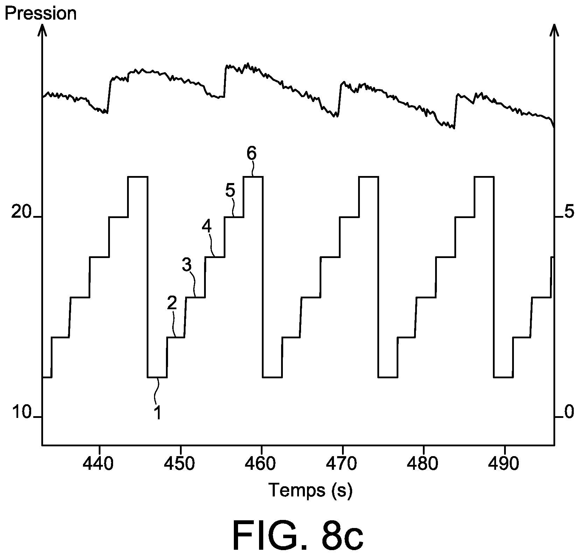

- s'il n'y pas de reflux, la pression, en début de phase 6, est légèrement supérieure à celle en début de phase 5. Le tissu cible absorbe donc bien le liquide injecté. Ce cas est illustré en figure 8B ;
- en cas de reflux, le liquide s'échappe du tissu et la pression en début de phase 6 est la même, voire inférieure, à celle en début de phase 5. Ce cas est illustré en figure 8C.

On peut donc introduire, pour l'exemple considéré, les variables suivantes :

- $P_6$: la pression mesurée pendant une durée de, par exemple, 1 seconde après le début de la phase 6,
- $P_5$: la pression mesurée pendant une durée de, par exemple, 1 seconde après le début de la phase 5.

On propose donc d'utiliser l'indicateur suivant pour évaluer la présence de reflux :

$$J = P_6 - P_5$$

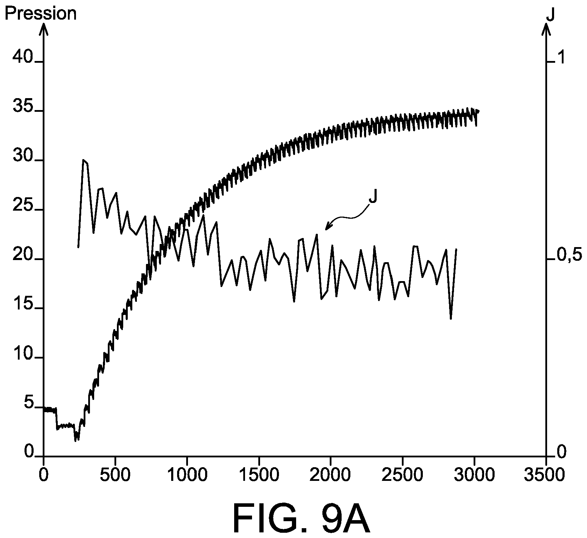

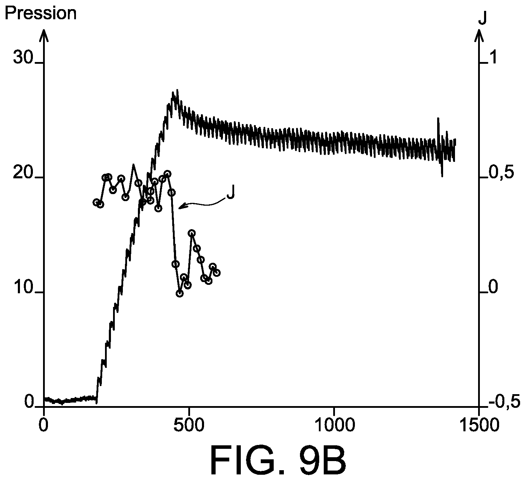

On a représenté l'évolution de ce paramètre sur les figures 9A et 9B (J étant mesuré une fois par cycle de fonctionnement de la pompe) :

- la figure 9A correspond au cas où il n'y a pas de reflux, les valeurs de J oscillent autour de 0.5 (échelle de droite) ; l'autre courbe représente l'évolution de la pression en fonction du temps;

- la figure 9B correspond au cas où il y a reflux, on observe alors une chute de la valeur de l'indicateur J qui peut être proche de 0 ou même négatif (échelle de droite). On comprend alors qu'en fixant un seuil $J_{threshold}$ inférieur à 0.5, par exemple compris entre 0 et 0.2 ($0 \leq J_{threshold} < 0,5$), on peut conclure à la présence d'un reflux lorsque $J \leq Jt_{hreshold}$.

Plus généralement, la mesure de la pression au cours des différentes phases de fonctionnement d'une pompe, notamment une pompe cyclique et en particulier une pompe péristaltique, permet de former un indicateur J, à partir de différentes mesures de pression, généralement au cours d'un même cycle, cet indicateur étant représentatif d'une situation de reflux. Cet indicateur peut être, comme dans l'exemple donné ci-dessus, la différence entre les pressions mesurées au cours des deux dernières phases du cycle de la pompe, ou, plus généralement, une différence entre les pressions mesurées au cours de deux phases différentes du cycle de la pompe. La comparaison de cet indicateur J avec un indicateur seuil $J_{threshold}$ prédéterminé peut alors permettre de conclure à la présence ou l'absence de reflux. On peut alors établir un indicateur correspondant à chaque cycle d'une série de cycles.

Les moyens 44, 46 de traitement des données peuvent, à partir des données fournies par la pompe 6, calculer le ou les paramètres en question, par exemple le paramètre J tel qu'indiqué ci-dessus. Ils peuvent également réaliser une comparaison de ce critère J ou de ce, ou de ces, paramètres à l'aide d'une valeur seuil $J_{threshold}$, pour, éventuellement, déclencher un arrêt de la pompe 4 ou un inversement de son fonctionnement. Enfin, les valeurs du paramètre retenu peuvent être présentées à un opérateur par les moyens de visualisation 47. Une situation de reflux peut également être signalée à celui-ci, par un signal visuel et/ou auditif ce que, là encore, les moyens 44,46 peuvent réaliser.

Dans une variante du système on emploie une micro-pompe comportant des vannes passives, c'est-à-dire des vannes non activables par un effecteur extérieur. De telles vannes s'ouvrent et se referment sous l'effet de la pression dans la pompe, le pompage étant réalisé par une vanne activée, par exemple par un moyen piézoélectrique. L'avantage en est une simplification de la technologie du système et donc du coût, mais c'est au détriment des performances (en termes de sécurité notamment). Il est alors en effet impossible de réduire la pression dans le circuit fluidique de la pompe, seul l'arrêt rapide de l'injection serait possible. De plus, ce type de vanne ne permet pas de fonctionner selon un mode réversible.

L'indicateur de reflux J peut être déterminé simplement, par exemple en comparant deux valeurs mesurées à deux instants différents, comme explicité ci-dessus. Mais, d'une façon générale, des indicateurs plus complexes peuvent être envisagés, à partir des valeurs de pression à différents instants.

L'invention peut être mise en œuvre dans le cadre de toute application nécessitant l'injection localisée, à l'aide d'un cathéter, d'un liquide, par exemple un liquide thérapeutique anti-tumoral, notamment dans un organe de structure peu compacte (par exemple analogue au cerveau).

Ainsi, on peut réaliser une insertion d'un cathéter dans un tissu 11 d'un patient (ou, plus généralement, d'un mammifère vivant), comme illustré sur chacune des figures 2A - 2C, ce cathéter ayant été, ou étant ensuite, relié à un ensemble comportant une pompe 4 et un capteur 6 tel que décrit ci-dessus, le tout pouvant être ensuite relié à un réservoir 2 d'un liquide à injecter dans le tissu 11. Ces étapes sont précédées d'une étape chirurgicale pour introduire le cathéter dans le tissu.

Un procédé d'injection et de contrôle de l'injection d'un flux de liquide à injecter, comporte :

- l'injection du fluide par une pompe péristaltique et un cathéter ;
- la mesure de la pression de liquide dans le cathéter à l'aide d'un capteur de pression ;
- le contrôle de la pompe en fonction des données mesurées par le capteur de pression à différents instants, ou en fonction de la valeur d'un indicateur, établi lui-même en fonction des mesures de la pression à différents instants d'un cycle de fonctionnement de la pompe.

Le liquide à injecter est par exemple un liquide médical tel qu'un liquide antitumoral.

Dans un tel procédé, il est possible de contrôler la pompe en fonction des données du capteur de pression ou de la valeur de l'indicateur.

La détermination de la présence ou l'absence d'un reflux peut résulter du calcul d'un indicateur établi en combinant au moins deux mesures de la pression à différents instants. Dans le cas d'une pompe cyclique, par exemple une pompe péristaltique, cet indicateur peut être calculé en combinant au moins deux pressions observées au cours d'un même cycle.

L'indicateur peut être déterminé en fonction de la comparaison de la pression mesurée à deux instants d'un même cycle, ou encore en fonction de la différence entre les pressions mesurées à deux instants d'un même cycle.

De préférence, indicateur est déterminé pour chaque cycle d'une série de cycles.

En fonction de la valeur de cet indicateur, les moyens de contrôle agissent automatiquement sur la pompe, notamment lorsque cette valeur indique l'apparition d'un phénomène de reflux.

La détection d'un reflux du liquide injecté permet d'inverser le sens de fonctionnement de la pompe lors de la détection d'un reflux du liquide injecté.

## Revendications

1. Système d'injection et de contrôle d'un flux de liquide à injecter, comportant:

   - un cathéter (8), comportant un conduit, pour délivrer un fluide à injecter,
   - une pompe péristaltique (4), ladite pompe fonctionnant par cycle, de telle sorte qu'un volume élémentaire de fluide est délivré au cours de chaque cycle,
- un capteur de pression (6), pour mesurer la pression de liquide qui s'écoule dans ledit conduit, depuis la pompe vers une extrémité du conduit,

**caractérisé en ce que** le système comporte en outre :

- des moyens de contrôle (44) pour recevoir des données du capteur de pression (6), et, pour chaque cycle d'une série de cycles de fonctionnement de ladite pompe, pour générer un indicateur, relatif à ce cycle, en fonction de la comparaison de ladite pression à deux instants différents de ce même cycle,
- les moyens de contrôle étant aptes à inverser la pompe en fonction de la valeur de cet indicateur.

2. Système selon la revendication 1, dans lequel le capteur de pression est disposé entre ladite pompe et l'extrémité dudit conduit, par laquelle est délivré le fluide.

3. Système selon l'une des revendications 1 ou 2, dans lequel les moyens de contrôle (44) sont aptes à générer un indicateur en fonction de la différence entre les pressions mesurées à deux instants d'un même cycle.

4. Système selon l'une des revendications précédentes, la pompe étant une micro-pompe péristaltique ou une micro-pompe à vannes passives.

5. Système selon l'une des revendications précédentes, comportant en outre des moyens de contrôle du débit, pour augmenter ou réduire le débit de la pompe en fonction de la valeur de l'indicateur.

6. Système selon l'une des revendications précédentes, un cycle de pompage comportant :

- une première phase, durant laquelle la pompe ne délivre pas de liquide dans le cathéter, la pression dans le cathéter étant stable en l'absence de reflux ;
- une deuxième phase, durant laquelle la pompe injecte le liquide dans le cathéter, induisant un saut de pression dans le cathéter, la présence d'un reflux se traduisant par un amoindrissement de ce saut de pression, par rapport à un saut de pression en l'absence de reflux.

**Patentansprüche**

1. System zur Injektion und zur Steuerung eines Flusses einer zu injizierenden Flüssigkeit, umfassend:

- einen Katheter (8), umfassend eine Leitung zum Liefern eines zu injizierenden Fluids,
- eine peristaltische Pumpe (4), wobei die Pumpe in Zyklen derart funktioniert, dass im Verlauf jedes Zyklus ein elementares Fluidvolumen geliefert wird,
- einen Drucksensor (6) zum Messen des Drucks der Flüssigkeit, die in der Leitung von der Pumpe in Richtung eines Endes der Leitung strömt,

**dadurch gekennzeichnet, dass** das System ferner umfasst:

- Steuerungsmittel (44) zum Empfangen von Daten vom Drucksensor (6), und zum Generieren, für jeden Zyklus einer Serie von Funktionszyklen der Pumpe, eines Indikators mit Bezug zu diesem Zyklus als Funktion des Vergleichs des Drucks bei zwei verschiedenen Zeitpunkten dieses gleichen Zyklus,
- wobei die Steuerungsmittel dazu ausgelegt sind, die Pumpe als Funktion des Werts dieses Indikators zu invertieren.

2. System nach Anspruch 1, bei dem der Drucksensor zwischen der Pumpe und dem Ende der Leitung angeordnet ist, durch die das Fluid geliefert wird.

3. System nach einem der Ansprüche 1 oder 2, bei dem die Steuerungsmittel (44) dazu ausgelegt sind, einen Indikator als Funktion der Differenz zwischen den Drücken zu generieren, die bei zwei Zeitpunkten ein und desselben Zyklus gemessen werden.

4. System nach einem der vorhergehenden Ansprüche, wobei die Pumpe eine peristaltische Mikropumpe oder eine Mikropumpe mit passiven Ventilen ist.

5. System nach einem der vorhergehenden Ansprüche, ferner umfassend Mittel zur Steuerung der Rate zum Vergrößern oder Verkleinern der Rate der Pumpe als Funktion des Werts des Indikators.

6. System nach einem der vorhergehenden Ansprüche, wobei ein Pumpzyklus umfasst:

- eine erste Phase, während der die Pumpe keine Flüssigkeit in den Katheter liefert, wobei der Druck in dem Katheter in Abwesenheit eines Rückflusses stabil ist;
- eine zweite Phase, während der die Pumpe die Flüssigkeit in den Katheter injiziert, was einen Drucksprung in dem Katheter induziert, wobei das Auftreten eines Rückflusses zu einer Verringerung dieses Drucksprung bezogen auf einen Drucksprung in Abwesenheit eines Rückflusses führt.

**Claims**

1. System for injecting and controlling a flow of liquid to be injected, comprising:

   - a catheter (8), comprising a duct, for delivering a fluid to be injected,
   - a peristaltic pump (4), said pump operating in cycles, such that an elementary volume of fluid is delivered during each cycle,
   - a pressure sensor (6), for measuring the liquid pressure which flows in said duct, from the pump to an end of the duct,

   **characterized in that** the system further comprises:

   - control means (44) for receiving data from the pressure sensor (6), and, for each cycle of a series of cycles, for generating an indicator, relative to this cycle, on the basis of at least two measurements of said pressure at different times of a same cycle,
   - the control means being able to reverse the operation of the pump on the basis of the value of this indicator.

2. System according to claim 1, in which the pressure sensor is arranged between said pump and the end of said duct, through which the fluid is delivered.

3. System according to claims 1 or 2, in which the control means (44) are able to generate an indicator on the basis of the difference between the pressures measured at two times of a same cycle.

4. System according to one of the preceding claims, the pump being a peristaltic micro-pump or a passive check valve micro-pump.

5. System according to one of the preceding claims, further comprising means of controlling the flow rate, to increase or to reduce the flow rate of the pump on the basis of the value of the indicator.

6. System according to one of the preceding claims, a pumping cycle comprising :

   - a first phase, during which the pump does not deliver liquid into the catheter, the pressure in the catheter being stable in the absence of reflux;

   - a second phase, during which the pump injects the liquid into the catheter, which induces a pressure jump in the catheter, the presence of a reflux resulting in a lessening of this pressure jump, compared to the pressure jump in the absence of reflux.

FIG. 1

FIG. 2A

FIG. 2B

FIG. 2C

EP 2 841 129 B1

FIG. 3

FIG. 4A

13

FIG. 4B

FIG. 5A

# FIG. 5B

FIG. 6

FIG. 7A

FIG. 7B

FIG. 7C

FIG. 7D

FIG. 7E

FIG. 7F

FIG. 7G

FIG. 8A

FIG. 8B

FIG. 8c

FIG. 9A

FIG. 9B

**RÉFÉRENCES CITÉES DANS LA DESCRIPTION**

**Documents brevets cités dans la description**

- US 7794443 B
- US 4534756 A
- EP 1980201 A
- US 2005075624 A
- US 20110257593 A
- US 20110144540 A
- US 20050145008 A
- WO 2011058140 A

**Littérature non-brevet citée dans la description**

- **PAUL F. MORRISON.** *Focal delivery during direct infusion to brain : role of flow rate, cathéter diameter and tissue mechanics*